# EUROPEAN PATENT SPECIFICATION

(11) **EP 1 075 283 B1**
(45) Date of publication and mention of the grant of the patent: **22.07.2009**
(21) Application number: 99918823.8
(22) Date of filing: 23.04.1999
(51) Int. Cl.: A61K 48/00, C12N 15/09

(54) **A PROCESS OF DELIVERING A POLYNUCLEOTIDE TO A CELL VIA THE VASCULAR SYSTEM**
VERFAHREN ZUR VERABREICHUNG EINES POLYNUKLEOTIDES AN EINE ZELLE ÜBER DAS VASKULARE SYSTEM
TECHNIQUE D'APPORT D'ACIDE NUCLEIQUE A UNE CELLULE PAR L'INTERMEDIAIRE DU SYSTEME VASCULAIRE

(30) Priority: 30.04.1998 US 70303
(43) Date of publication of application: 14.02.2001
(60) Divisional application: 08075946.7
(73) Proprietor: Mirus Bio Corporation, Madison, WI 53719 (US)
(72) Inventor: WOLFF, Jon, A., Madison, WI 53705 (US); KNECHTLE, Stuart, J., Oregon, WI 53575 (US); BUDKER, Vladimir, G., Madison, WI 53705 (US)
(74) Representative: Van Someren, Petronella F. H. M.
(86) International application number: PCT/US1999/008966
(87) International publication number: WO 1999/055379

(56) References cited:
- WO-A-98/58542
- WO-A1-94/11506
- WO-A1-97/26337
- US-A- 5 698 531
- ZHANG G ET AL: "EXPRESSION OF NAKED PLASMID DNA INJECTED INTO THE AFFERENT AND EFFERENT VESSELS OF RODENT AND DOG LIVERS" HUMAN GENE THERAPY, vol. 8, no. 15, 10 October 1997 (1997-10-10), pages 1763-1772, XP009004213 ISSN: 1043-0342
- BUDKER V ET AL: "Naked DNA delivered intraportally expresses efficiently in hepatocytes" GENE THERAPY, MACMILLAN PRESS LTD., BASINGSTOKE, GB, vol. 3, no. 7, 1996, pages 593-598, XP002201558 ISSN: 0969-7128
- BUDKER V ET AL: "THE EFFICIENT EXPRESSION OF INTRAVASCULARLY DELIVERED DNA IN RAT MUSCLE" GENE THERAPY, MACMILLAN PRESS LTD., BASINGSTOKE, GB, vol. 5, no. 2, February 1998 (1998-02), pages 272-276, XP002921644 ISSN: 0969-7128
- DORAN S E ET AL: "GENE EXPRESSION FROM RECOMBINANT VIRAL VECTORS IN THE CENTRAL NERVOUS SYSTEM AFTER BLOOD-BRAIN BARRIER DISRUPTION" NEUROSURGERY, WILLIAMS & WILKINS, BALTIMORE, MD, US, vol. 36, no. 5, May 1995 (1995-05), pages 965-970, XP009004222 ISSN: 0148-396X
- HERWEIJER H WOLFF J A: "Self-amplifying vectors for gene delivery" ADVANCED DRUG DELIVERY REVIEWS, AMSTERDAM, NL, vol. 27, 1997, pages 5-16, XP002959458 ISSN: 0169-409X
- ZHANG GUOFENG ET AL: "High levels of foreign gene expression in hepatocytes after tail vein injections of naked plasmid DNA" HUMAN GENE THERAPY, vol. 10, no. 10, 1 July 1999 (1999-07-01), pages 1735-1737, XP002201559 ISSN: 1043-0342
- CARDOSO et al., "In Situ Retrovirus-Mediated Gene Transfer Into Dog Liver", HUMAN GENE THERAPY, August 1993, Vol. 4, No. 4, pages 411-418, XP002921643.
- BUSKER et al., "The Efficient Expression of Intravascularly Delivered DNA in Rat Muscle", GENE THERAPY, February 1998, Vol. 5, No. 2, pages 272-276, XP002921644.
- LU et al., "Antisense DNA Delivery In Vivo: Liver Targeting by Receptor-Mediated Uptake", J. NUCLEAR MED., February 1994, Vol. 35, No. 2, pages 269-275, XP002921645.
- YANG, Y. ET AL: "Immune responses to viral antigens versus transgene product in the elimination of recombinant adenovirus-infected hepatocytes in vivo." GENE THERAPY, vol. 3, 1 January 1996 (1996-01-01), pages 137-144, XP008083572

## Description

### Field of the Invention

The invention generally relates to techniques for transferring genes into mammalian muscle parenchymal cells in vivo. More particularly, a method is provided for transfecting muscle parenchymal cells with polynucleotides delivered intravascularly.

### Background of the Invention

It was first observed that the in vivo injection of plasmid DNA into muscle enabled the expression of foreign genes in the muscle (Wolff, J A, Malone, R W, Williams, P, et al. Direct gene transfer into mouse muscle in vivo. Science 1990;247:1465-1468.). Since that report, several other studies have reported the ability for foreign gene expression following the direct injection of DNA into the parenchyma of other tissues. Naked DNA was expressed following its injection into cardiac muscle (Acsadi, G., Jiao, S., Jani, A., Duke, D., Williams, P., Chong, W., Wolff, J.A. Direct gene transfer and expression into rat heart in vivo. The New Biologist 3(1), 71-81, 1991.), pig epidermis (Hengge, U. R, Chan, E. F., Foster, R A., Walker, P. S., and Vogel, J. C. Nature Genetics 10:161-166 (1995)), rabbit thyroid (M. Sikes, B. O'Malley, M Finegold, and F. Medley, Hum. Gene Ther. 5, 837 (1994), lung by intratracheal injection (K B. Meyer, M M Thompson, M Y. Levy, L. G. Barron, F. C. Szoka, Gene Ther. Z, 450 (1995)), into arteries using a hydrogel-coated angioplasty balloon (R Riessen et al, Human Gene Ther. 4, 749 (1993)) (G. Chapman et al. Circ. Res. 71, 27 (1992)), melanoma tumors (R. G. Vile and L R Hart, Cancer Res. 53, 962 (1993)) and rat liver [(Malone, R W. et al. JBC 269:29903-29907 (1994)) (Hickman, M. A. Human Gene Therapy 5:1477-1483 (1994))].

Another important target tissue for gene therapy is the mammalian liver, given its central role in metabolism and the production of serum proteins. A variety of techniques have been developed to transfer genes into the liver. Cultured hepatocytes have been genetically modified by retroviral vectors [(Wolff, J. A. et al. PNAS 84:3344-3348 (1987) (Ledley, F. D., Darlington, G J., Hahn, T. and Woo, S.C.L. PNAS 84:5335-5339 (1987)] and re-implanted back into the livers in animals and in people [(J. R Chowdhury et al. Science 254, 1802 (1991) (M. Grossman et al. Nature Genetics 6, 335 (1994)]. Retroviral vectors have also been delivered directly to livers in which hepatocyte division was induced by partial hepatectomy [(Kay, MA. et al Hum Gene Ther. 3:641-647 (1992) (Ferry, N., Duplessis, O., Houssin, D., Danos, O. and Heard, J.-M PNAS 88:8377-8381 (1991) (Kaleko, M, Garcia, J.V. and Miller, A.D. Hum Gene THer. 2:27-32 (1991)]. The injection of adenoviral vectors into the portal or systemic circulatory systems leads to high levels of foreign gene expression that is transient [(L. D. Stratford-Perricaudet, M Levrero, J. F. Chasse, M Perricaudet, P. Briand, Hum. Gene Ther. 1, 241 (1990) (H. A. Jaffe et al. Nat. Genet. 1, 372 (1992) (Q. Li, M A. Kay, M Finegold, L. D. Stratford-Pemcaudet, S. L. C. Woo, Hum. Gene Ther. 4, 403 (1993)]. Non-viral transfer methods have included polylysine complexes of asialoglycoproteins that are injected into the system circulation [Wu, G. Y. and Wu, C. H J. Biol. Chem. 263:14621-14624 (1988)].

Foreign gene expression has also been achieved by repetitively injecting naked DNA in isotonic solutions into the liver parenchyma of animals treated with dexamethasone [(Malone, R. W. et al. JBC 269:29903-29907 (1994) (Hickman, M A. Human Gene Therapy 5:1477-1483 (1994)]. Plasmid DNA expression in the liver has also been achieved via liposomes delivered by tail vein or intraportal routes [(Kaneda, Y., Kunimitsu, L and Uchida, T. J. Biol. Chem. 264:12126-12129 (1989) (Soriano, P. et al. PNAS 80:7128-7131 (1983) Kaneda, Y., Iwai, K. and Uchida, T. Science 243:375-378 (1989)].

Regarding transformation of muscle cells, WO 94/11506, in the name of Arch Development, describes adenovirus-mediated gene transfer to cardiac and vascular smooth muscle by infusing an adenovirus vector into a perfusing blood vessel. US 5,698,531, in the name of the Reagents of the University of Michigan, describes the delivery of recombinant genes in cells by supplying the circulation of the heart through a catheter. WO 97/26337, in the name of Avigen, describes methods for delivering DNA to muscle cells by intramuscular injection of recombinant adeno-associated virus. Budker et al., Gene Therapy 5, 272-276, describes the efficient expression of intravascularly delivered plasmid DNA in rat muscle.

Despite this progress, there is still a need for a gene transfer method that can efficiently and safely cause expression of foreign genes in muscle cells.

### Summary of The Invention

The present invention provides for the transfer of polynucleotides into parenchymal cells within muscle tissues in situ and in vivo. An intravascular route of administration enables a prepared polynucleotide to be delivered to the parenchyma cells more evenly distributed and more efficiently expressed than direct parenchymal injections. The efficiency of polynucleotide delivery and expression was increased substantially by increasing the permeability of the tissue's blood vessel. This was done by increasing the intravascular hydrostatic (physical) pressure.

A process is described for delivering a polypeptide into a muscle parenchymal cell in claim 1.

### Brief Description of the Drawings

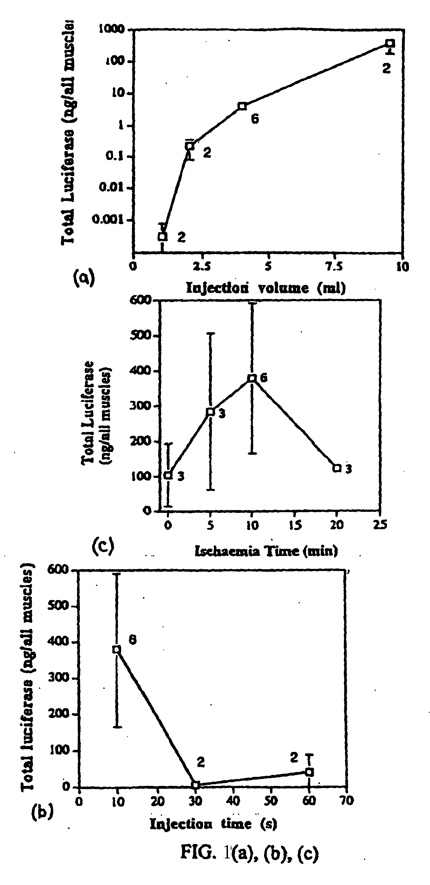

**Fig. 1(a), (b), (c):** Effects of injection solution volume (a), injection time (b) and preinjection eschemia time (c) on the mean levels of luciferase expression in rat hindlimb muscles. Two days after 475 ug of pCILux in 9.5 ml of NSS were injected unilaterally into the iliac artery of adult Sprague-Dawley rats, luciferase activities in 6 hindlimb muscle groups (anterior upper leg, medial upper leg, posterior upper leg, anterior lower leg, posterior lower leg and foot) were measured as previously reported and presented as the means of the total luciferase levels of all 6 muscle groups. The pre-injection ischemia time was 10 min in "a" and "b". Numbers adjacent to data points indicate the number of animals (1 limb/animal) assayed for each condition. Error bars indicate the standard error.

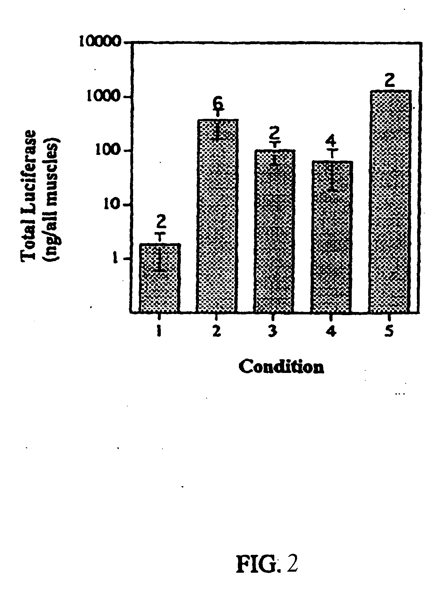

**Fig. 2****:** Effects of various conditions on luciferase expression in rat hind limbs muscles following the injection of pCILux DNA into the iliac artery. Condition 1,475 ug DNA in 9.5 ml of NSS were injected within 10 sec after a 10 min occlusion of limb blood flow, Condition 2, 475 ug DNA in 9.5 ml of H2O were injected within 10 sec after a 10 min occlusion of limb blood flow; Condition 3, 475 ug DNA in 9.5 ml of NSS with 15% mannitol were within 30 sec after a 10 min occlusion of limb blood flow; Condition 4, 80 ug of collagenase in 1 ml ofNSS were injection into iliac artery immediately after blood flow occlusion. After 10 min, blood flow was unclosed for several sec, closed again, and 475 ug DNA in 9.5 ml of NSS were injected within 10 sec. Bold numbers indicate the number of animals assayed at each experimental conditions. Error bars indicate the standard error.

### Detailed Description

### A. Definitions

The term, naked polynucleotides, indicates that the polynucleotides are not associated with a transfection reagent or other delivery vehicle that is required for the polynucleotide to be delivered to the parenchymal cell. A transfection reagent is a compound or compounds used in the prior art that bind(s) to or complex(es) with polynucleotides and mediates their entry into cells. The transfection reagent also mediates the binding and internalization of polynucleotides into cells. Examples of transfection reagents include cationic liposomes and lipids, calcium phosphate precipitates, and polylysine complexes. Typically, the transfection reagent has a net positive charge that binds to the polynucleotide's negative charge. The transfection reagent mediates binding of polynucleotides to cell via its positive charge (that binds to the cell membrane's negative charge) or via ligands that bind to receptors in the cell. For example, cationic liposomes or polylysine complexes have net positive charges that enable them to bind to DNA. Other vehicles are also used, in the prior art, to transfer genes into cells. These include complexing the polynucleotides on particles that are then accelerated into the cell. This is termed biolistic or gun techniques. Other methods include eletroporation in which a device is used to give an electric charge to cells. The charge increases the permeability of the cell.

The term polynucleotide is a term of art that refers to a string of at least two base-sugar-phosphate combinations. Nucleotides are the monomeric units of nucleic acid polymers. The term includes deoxyribonucleic acid (DNA) and ribonucleic acid (RNA) in the form of an oligonucleotide messenger RNA, anti-sense, plasmid DNA, parts of a plasmid DNA or genetic material derived from a virus. A polynucleotide is distinguished, here, from a oligonucleotide by containing more than 120 monomeric units. Anti-sense is a polynucleotide that interferes with the function of DNA and/or RNA. A polynucleotide is considered in this specification to include a non-natural polynucleotide (not occurring in nature), for example: a derivative of natural nucleotides such as phosphothionates or peptide nucleic acids.

A polynucleotide can be delivered to a cell in order to produce a cellular change that is therapeutic. The delivery of polynucleotides or other genetic material for therapeutic purposes (the art of improving health in an animal including treatment or prevention of disease) is gene therapy. The polynucleotides are coded to express a whole or partial protein, or may be anti-sense, and can be delivered either directly to the organism in situ or indirectly by transfer to a cell that is then transplanted into the organism. The protein can be missing or defective in an organism as a result of genetic, inherited or acquired defect in its genome.

For example, a polynucleotide may be coded to express the protein dystrophin that is missing or defective in Duchenne muscular dystrophy. The coded polynucleotide is delivered to a selected group or groups of cells and incorporated into those cell's genome or remain apart from the cell's genome. Subsequently, dystrophin is produced by the formerly deficient cells. Other examples of imperfect protein production that can be treated with gene therapy include the addition of the protein clotting factors that are missing in the hemophilias and enzymes that are defective in inborn errors of metabolism such as phenylalanine hydroxylase.

A delivered polynucleotide can also be therapeutic in acquired disorders such as neurodegenerative disorders, cancer, heart disease, and infections. The polynucleotide has its therapeutic effect by entering the cell. Entry into the cell is required for the polynucleotide to produce the therapeutic protein, to block the production of a protein, or to decrease the amount of a RNA.

Additionally, a polynucleotide can be delivered to block gene expression. Such polynucleotides can be anti-sense by preventing translation of a messenger RNA or could block gene expression by preventing transcription of the gene. Preventing both RNA translation as well as DNA transcription is considered preventing expression. Transcription can be blocked by the polynucleotide binding to the gene as a duplex or triplex. It could also block expression by binding to proteins that are involved in a particular cellular biochemical process.

Polynucleotides may be delivered that recombine with genes. The polynucleotides may be DNA, RNA, hybrids and derivatives of natural nucleotides. Recombine is the mixing of the sequence of a delivered polynucleotide and the genetic code of a gene. Recombine includes changing the sequence of a gene.

Delivery of a polynucleotide means to transfer a polynucleotide from a container outside a mammal to within the outer cell membrane of a cell in the mammal. The term transfection is used herein, in general, as a substitute for the term delivery, or, more specifically, the transfer of a polynucleotide from directly outside a cell membrane to within the cell membrane. If the polynucleotide is a primary RNA transcript that is processed into messenger RNA, a ribosome translates the messenger RNA to produce a protein within the cytoplasm. If the polynucleotide is a DNA, it enters the nucleus where it is transcribed into a messenger RNA that is transported into the cytoplasm where it is translated into a protein. The polynucleotide contains sequences that are required for its transcription and translation. These include promoter and enhancer sequences that are required for initiation. DNA and thus the corresponding messenger RNA (transcribed from the DNA) contains introns that must be spliced, poly A addition sequences, and sequences required for the initiation and termination of its translation into protein. Therefore if a polynucleotide expresses its cognate protein, then it must have entered a cell.

A therapeutic effect of the protein in attenuating or preventing the disease state can be accomplished by the protein either staying within the cell, remaining attached to the cell in the membrane or being secreted and dissociating from the cell where it can enter the general circulation and blood. Secreted proteins that can be therapeutic include hormones, cytokines, growth factors, clotting factors, anti-protease proteins (e.g. alpha-antitrypsin) and other proteins that are present in the blood Proteins on the membrane can have a therapeutic effect by providing a receptor for the cell to take up a protein or lipoprotein. For example, the low density lipoprotein (LDL) receptor could be expressed in hepatocytes and lower blood cholesterol levels and thereby prevent atherosclerotic lesions that can cause strokes or myocardial infarction. Therapeutic proteins that stay within the cell can be enzymes that clear a circulating toxic metabolite as in phenylketonuria. They can also cause a cancer cell to be less proliferative or cancerous (e.g. less metastatic). A protein within a cell could also interfere with the replication of a virus.

The delivered polynucleotide can stay within the cytoplasm or nucleus apart from the endogenous genetic material. Alternatively, the polynucleotide could recombine (become a part of) the endogenous genetic material. For example, DNA can insert into chromosomal DNA by either homologous or non-homologous recombination.

Parenchymal cells are the distinguishing cells of a gland or organ contained in and supported by the connective tissue framework. The parenchymal cells typically perform a function that is unique to the particular organ. The term "parenchymal" often excludes cells that are common to many organs and tissues such as fibroblasts and endothelial cells within the blood vessels.

In a liver organ, the parenchymal cells include hepatocytes, Kupffer cells and the epithelial cells that line the biliary tract and bile ductules. The major constituent of the liver parenchyma are polyhedral hepatocytes (also known as hepatic cells) that presents at least one side to an hepatic sinusoid and apposed sides to a bile canaliculus. Liver cells that are not parenchymal cells include cells within the blood vessels such as the endothelial cells or fibroblast cells.

In striated muscle, the parenchymal cells include myoblasts, satellite cells, myotubules, and myofibers. In cardiac muscle, the parenchymal cells include the myocardium also known as cardiac muscle fibers or cardiac muscle cells and the cells of the impulse connecting system such as those that constitute the sinoatrial node, amoventricular node, and atrioventricular bundle.

In a pancreas, the parenchymal cells include cells within the acini such as zymogenic cells, centroacinar cells, and basal or basket cells and cells within the islets of Langerhans such as alpha and beta cells.

In spleen, thymus, lymph nodes and bone marrow, the parenchymal cells include reticular cells and blood cells (or precursors to blood cells) such as lymphocytes, monocytes, plasma cells and macrophages.

In the nervous system which includes the central nervous system (the brain and spinal cord) peripheral nerves, and ganglia, the parenchymal cells include neurons, glial cells, microglial cells, oligodendrocytes, Schwann cells, and epithelial cells of the choroid plexus.

In the kidney, parenchymal cells include cells of collecting tubules and the proximal and distal tubular cells. In the prostate, the parenchyma includes epithelial cells.

In glandular tissues and organs, the parenchymal cells include cells that produce hormones. In the parathyroid glands, the parenchymal cells include the principal cells (chief cells) and oxyphilic cells. In the thyroid gland, the parenchymal cells include follicular epithelial cells and parafollicular cells. In the adrenal glands, the parenchymal cells include the epithelial cells within the adrenal cortex and the polyhedral cells within the adrenal medulla

In the parenchyma of the gastrointestinal tract such as the esophagus, stomach, and intestines, the parenchymal cells include epithelial cells, glandular cells, basal, and goblet cells.

In the parenchyma of lung, the parenchymal cells include the epithelial cells, mucus cells, goblet cells, and alveolar cells.

In fat tissue, the parenchymal cells include adipose cells or adipocytes. In the skin, the parenchymal cells include the epithelial cells of the epidermis, melanocytes, cells of the sweat glands, and cells of the hair root.

In cartilage, the parenchyma includes chondrocytes. In bone, the parenchyma includes osteoblasts, osteocytes, and osteoclasts.

An intravascular route of administration enables a polynucleotide to be delivered to parenchymal cells more evenly distributed and more efficiently expressed than direct parenchymal injections. Intravascular herein means within a hollow tubular structure called a vessel that is connected to a tissue or organ within the body. Within the cavity of the tubular structure, a bodily fluid flows to or from the body part. Examples of bodily fluid include blood, lymphatic fluid, or bile. Examples of vessels include arteries, arterioles, capillaries, venules, sinusoids, veins, lymphatics, and bile ducts. The intravascular route includes delivery through the blood vessels such as an artery or a vein.

Polypeptide refers to a linear series of amino acid residues connected to one another by peptide bonds between the alpha-amino group and carboxy group of contiguous amino acid residues.

Protein refers to a linear series of greater than 50 amino acid residues connected one to another as in a polypeptide.

Vectors are polynucleic molecules originating from a virus, a plasmid, or the cell of a higher organism into which another nucleic fragment of appropriate size can be integrated without loss of the vectors capacity for self replication; vectors introduce foreign DNA into host cells, where it can be reproduced. Examples are plasmids, cosmids, and yeast artificial chromosomes; vectors are often recombinant molecules containing DNA sequences from several sources. A vector includes a viral victor for example, adenovirus (icosahedral (20-sided) virus that contains; there are over 40 different adenovirus varieties, some of which cause the common cold) DNA; adenoassociated viral vectors (AAV) which are derived from adenoassociated viruses and are smaller than adenoviruses; and retrovirus (any virus in the family Retroviridae that has RNA as its nucleic acid and uses the enzyme reverse transcriptase to copy its genome into the DNA of the host cell's chromosome; examples include VSV G and retroviruses that contain components of lentivirus including HIV type viruses).

Afferent blood vessels of organs are defined as vessels which are directed towards the organ or tissue and in which blood flows towards the organ or tissue under normal physiologic conditions. Conversely, the efferent blood vessels of organs are defined as vessels which are directed away from the organ or tissue and in which blood flows away from the organ or tissue under normal physiologic conditions. In the liver, the hepatic vein is an efferent blood vessel since it normally carries blood away from the liver into the inferior vena cava Also in the liver, the portal vein and hepatic arteries are efferent blood vessels in relation to the liver since they normally carry blood towards the liver.

### B. Delivery of Polynucleotides

A needle or catheter is used to inject the polynucleotide into the vascular system The injection can be performed under direct observation following an incision and visualization of the tissues blood vessels. Alternatively, a catheter can be inserted at a distant site and threaded so that it resides in the vascular system that connects with the target tissue. In another embodiment, the injection could be performed by using a needle that traverses the intact skin and enters a vessel that supplies or drains from the target tissue.

### C. Permeability

The efficiency of the polynucleotide delivery and expression was increased substantially by increasing the permeability of a blood vessel within the target tissue. Permeability is defined here as the propensity for macromolecules such as polynucleotides to move through vessel walls and enter the extravascular space. One measure of permeability is the rate at which macromolecules move through the vessel wall and out of the vessel. Another measure of permeability is the lack of force that resists the movement through the vessel wall and out of the vessel. Vessels contain elements that prevent macromolecules from leaving the intravascular space (internal cavity of the vessel). These elements include endothelial cells and connective material (e.g. collagen). High permeability indicates that there are fewer of these elements that can block the egress of macromolecules and that the spaces between these elements are larger and more numerous. In this context, high permeability enables a high percentage of polynucleotides being delivered to leave the intravascular space; while low permeability indicates that a low percentage of the polynucleotides will leave the intravascular space.

The permeability of a blood vessel can be increased by increasing the intravascular hydrostatic pressure. The intravascular hydrostatic pressure is increased by rapidly (from 10 seconds to 30 minutes) injecting a polynucleotide in solution into the blood vessel which increases the hydrostatic pressure. Hydrostatic pressure is further increased by obstructing the outflow of the injection solution from the tissue for a period of time sufficient to allow delivery of a polynucleotide. Obstructing means to block or impede the outflow of injection fluid, thereby transiently (reversibly) blocking the outflow of the blood. For example, an afferent vessel supplying an organ is rapidly injected and the efferent vessel draining the tissue is ligated transiently. The efferent vessel (also called the venous outflow or tract) draining outflow from the tissue is also partially or totally clamped for a period of time sufficient to allow delivery of a polynucleotide. In the reverse, an efferent is injected and an afferent vessel is occluded.

The intravascular pressure of a blood vessel can also be increased by increasing the osmotic pressure within the blood vessel. Typically, hypertonic solutions containing salts such as NaCl, sugars or polyols such as mannitol are used. Hypertonic means that the osmolality of the injection solution is greater than physiologic osmolality. Isotonic means that the osmolality of the injection solution is the same as the physiological osmolality (the tonicity or osmotic pressure of the solution is similar to that of blood). Hypertonic solutions have increased tonicity and osmotic pressure similar to the osmotic pressure of blood and cause cells to shrink.

The permeability of the blood vessel can also be increased by a biologically-active molecule. A biologically-active molecule is a protein or a simple chemical such as histamine that increases the permeability of the vessel by causing a change in function, activity, or shape of cells within the vessel wall such as the endothelial or smooth muscle cells. Typically, biologically-active molecules interact with a specific receptor or enzyme or protein within the vascular cell to change the vessel's permeability. Biologically-active molecules include vascular permeability factor (VPF) which is also known as vascular endothelial growth factor (VEGF). Another type of biologically-active molecule can also increase permeability by changing the extracellular connective material. For example, an enzyme could digest the extracellular material and increase the number and size of the holes of the connective material.

### EXAMPLES

The following examples are intended to illustrate, but not limit, the present invention.

### Example 1

The intravascular delivery of naked pDNA to muscle cells also has attraction. Muscle has a high density of capillaries (Browning, 1996) and the capillaries are in close contact with the myofibers (Lee 1995). However, the endothelium in muscle capillaries is of the continuous, non-fenestrated type and has low solute permeability especially to large macromolecules (Taylor, A.E., and D.N.Granger. Exchange of macromolecules across the microcirculation. In: Handbook of Phusiology. The Cardiovascular System Microcirculation Bethesda, MD: Am. Physiol.Soc., 1984, sect 2, vol. IV, chapt 11, p. 467) The mechanism of macromolecule transendothelial transport is poorly understood. Cell biologists have proposed that this transport occurs by transcytosis involving plasmalemmal vesicles or convective transport through transient transendothelial channels formed by the fusion of vesicles. (Michel C.C. Cardiovascular Res. 1996). Physiologists have modeled the muscle endothelium as having a large number of small pore with radii of 4 nm and a very low number of large pores with radii of 20-30 nm.(Rippe B. Physiological Rev, 1994). Although the radius of gyration of 6 kb pDNA is ~100 nm (Fishman, D.M, Biopolymers, 38, 535-552), supercoiled DNA in plectonomic form has superhelix dimensions of approximately 10 nm (Rybenkov V.V. J. Mol. Biol. 267, 299-311 1997). This implies that pDNA has a possibility to traverse microvascular walls by stringing through their large pores. We hypothesized that the rate of pDNA extravasation could be increased by enhancing fluid convection through these large pores by raising the transmural pressure difference in selective regions. This report demonstrates that intravascular pDNA injections under high pressure can in fact lead to high levels of foreign gene expression in muscles throughout the selected hindlimb of an adult rat

### Hydrostatic Pressure

475 ug of pCELux in normal saline solution (NSS) was injected into the femoral arteries of adult Sprague-Dawley rats while blood inflow and outflow were blocked Injection of pCILux; a luciferase expression vector utilizing the CMV promoter (LIVER II promoter accepted for HGT), was done after both the femoral artery and vein was occluded for 10 min. Two days after pDNA injections, the luciferase activities were measured in all the muscles of the hindlimb (Fig. 5). The highest levels of luciferase expression were achieved when the pCILux was injected in 9.5 ml of normal saline within 10 sec. Injection volumes less than 9.5 ml resulted in substantially less expression (Fig. 5(a)). Injection times more than 10 sec also resulted in much less expression (Fig. 5(b)). This critical dependence on volume and speed of injection indicates that either increased hydrostatic pressure or rapid flow is required for efficient expression. Artery injections performed without occluding the femoral vein resulted in approximately 200-fold less expression (1.8±1.2 ng of luciferase per all hindlimb muscles).

### Other Factors

Further studies were performed to determine the effect of ischemia on the expression of the intravascularly injected pCILux. The time of ischemia was adjusted by varying the time that both the femoral artery and vein were occluded prior to pDNA injection (Fig. 5(c)). Although the highest level of expression was obtained with 10 min of ischemia, the expression levels were only a few-fold lower at shorter or longer ischemia times. This suggests that ischemia is not a critical factor for enabling egress of the pDNA out of the intravascular space. However, the blood flow for the zero ischemia time point is disrupted for ~30 sec and this may be sufficient to affect vascular permeability. Ischemia could increase expression either by capillary recruitment and vasodilatation) or augmenting permeability (Mathieu-Costello O. Int J Microcirc 1995.15, 231-237). In addition, ischemia could possibly increase pDNA expression by affecting transcription or translation. Ischemia can be tolerated by muscle for two to three hours (Gidlof A.,lnt. J. Microcirc. Clin. Exp. 1987, 7,67-86). Histologic analysis of the muscle did not reveal any hemorrhage or myofiber damage.
Other factors were explored to increase the level of expression. Hypotonic (Fig. 6, condition 2) or hypertonic (Fig. 6, condition 3) injection solutions resulted in less expression. Although, the effect of the hypertonic injection solution (normal saline solution with 15% mannitol) may have been mitigated by the slower rate of injection (over 30 sec instead of 10 sec) that results from its increased viscosity. The pre-injection with collagenase resulted in a 3.5-fold increase to 1,332 ng/total muscles (Fig. 6, condition 4). The collagenase was used to disrupt the basement membrane of the capillaries and thereby increase pDNA egress. It could have also increased expression by disrupting the extracellular matrix within the muscle. Further studies are in progress to determine the optimal conditions for collagenase administration without causing hemorrhage.

### Distribution of Foreign Gene Expression

The luciferase expression was distributed among all muscle groups in the leg (Table 1). The lower levels in the lower anterior leg and foot are probably due to their high content of tendons and small amount of muscles. The levels after intravascular injection was up to 40 times higher than the levels after intramuscular injection.

The type and percentage of the transfected cells in the muscle were determined using the β-galactosidase reporter system. Using the best injection condition (condition 4 in Fig. 6), approximately 10-50% of the myofibers expressed β-galactosidase.

These expression results provide indirect evidence that pDNA extravasation occurred. More direct evidence was obtained using fluorescently-labeled DNA injected into the femoral artery (condition I in Fig. 6). The labeled DNA was distributed extravascularly in all the limb muscles and surrounded most of the myofibers (data not shown).

### Conclusion

These results demonstrate that the intraarterial delivery of pDNA to muscle can be greatly enhanced when injected rapidly, in a large volume and when all blood vessels leading into and out of the hindlimb are occluded. These conditions presumably increase the intravascular hydrostatic pressure and thereby increase the convective outflow that transports the pDNA into contact with myofibers. The high intravascular pressure may increase the number, size and permeability of the microvascular pores (Rippe, B. Acta Physiol. Scand. 125, 453-459, 1985) (Wolf, M.B. Am. J. Physiol. 257, H2025-H2032,1989). Preliminary studies using collagenase suggest that enzymatic disruption of the vessels basement membrane or muscle extracellular matrix may also increase the delivery of pDNA to the myofibers. Ischemia also increased expression moderately. Furthermore, it is expected that other enzymes such as hylaronidase will perform similarly to collagenase.

This study shows that the intravascular approach increases expression in muscle up to 40-fold as compared to intramuscular injection. This is the first demonstration that naked plasmid DNA can be efficiently expressed in muscles of adult animals larger than mice. It is also the first report of an intravascular non-viral gene transfer approach for muscle. Further studies in larger animals will determine the clinical relevance of this study.

### Description of Injections

A 4 cm long abdominal midline excision was performed in 150-200 g, adult Sprague-Dawley rats anesthetized with 80 mg/mg ketamine and 40 mg/kg xylazine. Microvessel clips (Edward Weck, Inc., Research Triangle Park, NC) were placed on external iliac, caudal epigastric, internal iliac and deferent duct arteries and veins to block both outflow and inflow af the blood to the leg. A 27 g butterfly needle was inserted into the external iliac artery and the DNA solution was injected by hand.

**Table 1. Distribution of luciferase expression among the different hindlimb muscle groups two days following intraarterial injection with 475 µg of pCILux in 9.5 ml of NSS over 10. Means and their standard deviations are shown for 6 animals.**

| Injection type | Muscle group | Total Luciferase (ng) | Luciferase Conc. (ng/g tissue) |
|---|---|---|---|
| Intravascular | Upper Leg Anterior | 149.1± 51.2 | 109.2 ± 37.5 |
| | Upper Leg Posterior | 74.6 ± 36.0 | 43.4 ± 20.9 |
| | Upper Leg Medial | 88.7 ± 63.7 | 61.6 ± 44.5 |
| | Lower Leg Posterior | 114.5 ± 89.7 | 66.0 ± 51.7 |
| | Lower Leg Anterior | 3.0 ± 1.0 | 5.4 ± 1.7 |
| | Foot | 0.5 ± 0.2 | 4.1 ± 1.4 |
| | | | |
| Intramuscular | Upper Leg Anterior | 3.7 ± 0.9 | 2.8 ± 0.8 |

### Example 2

Adenoviral vectors can be delivered to muscle parenchymal cells by an intravascular route.

Methods: the same methods that were used to deliver naked plasmid DNA to muscle in rats were also used. The adenoviral vector CMVLacZ that expresses the E. coli beta-galactosidase from the immediate early promoter of the human cytomegalovirus (commonly called the "CMV promoter") was prepared as previously described (Yang, T., K. U. Jooss, Q. Su, H.C. J. Ertl and J.M. Wilson: Immune responses to viral antigens versus transgene product in the elimination of recombinant adenovirus-infected hepatocytes in vivo, Gene Therapy, 3(2): 137-144, 1996) The rat iliac artery was preinjected with 0.5 mg of papaverine and 40 ng of collagenase in 3 ml of saline while blocking the iliac artery and vein. 5 X 10⁸ particles of the adenoviral vector CMVLacZ in 10 ml of saline was injected in about 10 sec and 2 minutes later the clamps were opened. Two days after injection the leg muscle were assayed for luciferase as above.

### Results:

Table: Distribution of luciferase activity following the intraarterial injection of adenovirus CMVLacZ.

| **Muscle Group** | **Luciferase (ng)** |
|---|---|
| Upper Leg Anterior | 59.04 |
| Upper Leg Posterior | 18.33 |
| Upper Leg Medial | 4.44 |
| Lower Leg Posterior | 11.04 |
| Lower Leg Anterior | 5.33 |
| Foot | 0.22 |
| Total | 98.40 |

The foregoing is considered as illustrative only of the principles of the invention. Further, since numerous modifications and changes will readily occur to those skilled in the art, it is not desired to limit the invention to the exact construction and operation shown and described.

## Claims

1. Use of a viral vector for the preparation of a pharmaceutical composition for the treatment or prophylaxis of a mammal by gene therapy of muscle parenchymal cells within a limb, **characterized in that** the gene therapy is effected by obstructing the inflow and outflow of a blood vessel of said limb and inserting said viral vector into said blood vessel, said inserting comprises injecting said viral vector selected from the group consisting of adenoviral, adeno-associated viral and retroviral in solution from 10 seconds to 30 minutes into said blood vessel thereby increasing the hydrostatic pressure on the wall of said blood vessel and wherein said obstruction is for a period of time sufficient to allow delivery of the viral vector.

2. Use according to claim 1, wherein said blood vessel is selected from the group consisting of afferent and efferent vessels.

## Patentansprüche

1. Verwendung eines viralen Vektors zur Herstellung einer pharmazeutischen Zusammensetzung zur Behandlung oder Prophylaxe eines Säugetiers durch Gentherapie von parenchymatösen Muskelzellen innerhalb einer Extremität, **dadurch gekennzeichnet, dass** die Gentherapie bewirkt wird durch Obstruktion des Zuflusses und Abflusses eines Blutgefäßes der Extremität und Einsetzen des viralen Vektors in das Blutgefäß, wobei das Einsetzen aufweist Injizieren des viralen Vektors, ausgewählt aus der Gruppe bestehend aus Adenovirus, adenoassoziierter Virus und Retrovirus, in Lösung, von 20 Sekunden bis 30 Minuten lang in das Blutgefäß, wodurch der hydrostatische Druck auf die Wand des Blutgefäßes erhöht wird und wobei die Obstruktion für eine Zeitspanne erfolgt, die für das Zuführen des viralen Vektors ausreichend ist.

2. Verwendung nach Anspruch 1, wobei das Blutgefäß aus der Gruppe bestehend aus afferenten und efferenten Gefäßen ausgewählt ist.

## Revendications

1. Utilisation d'un vecteur viral pour la préparation d'une composition pharmaceutique destinée au traitement ou à la prophylaxie d'un mammifère par thérapie génique de cellules de parenchyme musculaire se trouvant dans un membre, **caractérisée en ce que** la thérapie génique est réalisée en bloquant l'écoulement d'entrée et de sortie d'un vaisseau sanguin dudit membre et en insérant ledit vecteur viral dans ledit vaisseau sanguin, ladite insertion comprenant l'injection dudit vecteur viral choisi dans le groupe constitué par un vecteur adénoviral, un vecteur viral adéno-associé et un vecteur rétroviral en solution pendant 10 secondes à 30 minutes dans ledit vaisseau sanguin de sorte à augmenter la pression hydrostatique sur la paroi dudit vaisseau sanguin, et ledit blocage étant réalisé pendant une durée suffisante pour permettre la distribution du vecteur viral.

2. Utilisation selon la revendication 1, dans laquelle ledit vaisseau sanguin est choisi dans le groupe constitué par les vaisseaux afférents et efférents.
